# EUROPÄISCHE PATENTSCHRIFT

(11) **EP 0 969 871 B1**
(45) Veröffentlichungstag und Bekanntmachung des Hinweises auf die Patenterteilung: **24.09.2003**
(21) Anmeldenummer: 98917090.7
(22) Anmeldetag: 26.03.1998
(51) Int. Cl.: A61K 47/24, A61K 9/14

(54) **VERFAHREN ZUR HERSTELLUNG VON HOMOGENEN, GLYCEROPHOSPHOLIPIDE UND POLARE ODER LIPOPHILE SUBSTANZEN ENTHALTENDEN, WASSERFREIEN FORMULIERUNGEN**
METHOD FOR THE PRODUCTION OF HOMOGENOUS WATER-FREE FORMULATIONS CONTAINING GLYCEROPHOSPHOLIPIDS AND POLAR OR LIPOPHILIC SUBSTANCES
PROCEDE DE PRODUCTION DE FORMULATIONS ANHYDRES HOMOGENES CONTENANT DES GLYCEROPHOSPHOLIPIDES ET DES SUBSTANCES POLAIRES OU LIPOPHILES

(30) Priorität: 27.03.1997 DE 19713093; 27.03.1997 DE 19713094
(43) Veröffentlichungstag der Anmeldung: 12.01.2000
(73) Patentinhaber: Degussa AG, 83308 Trostberg (DE)
(72) Erfinder: HEIDLAS, Jürgen, D-83308 Trostberg (DE); ZIRZOW, Karl-Heinz, D-83308 Trostberg (DE); WIESMÜLLER, Johann, D-84518 Garching (DE); GRAEFE, Jürgen, D-83308 Trostberg (DE)
(74) Vertreter: Weiss, Wolfgang, Dipl.-Chem. Dr.
(86) Internationale Anmeldenummer: EP9801789
(87) Internationale Veröffentlichungsnummer: WO98043674

(56) Entgegenhaltungen:
- EP-A- 0 051 833
- EP-A- 0 556 392
- WO-A-96/11669
- DE-A- 4 407 933
- DATABASE WPI Section Ch, Week 8727 Derwent Publications Ltd., London, GB; Class B05, AN 87-187991 XP002076711 & JP 62 116516 A (NIPPON OILS & FATS CO LTD), 28. Mai 1987 & PATENT ABSTRACTS OF JAPAN vol. 011, no. 332 (C-455) & JP 62 116516 A (NIPPON OILS & FATS CO LTD), 18. November 1985
- HEIDLAS J E: "PROPANE EXTRACTION IN FOOD PROCESSING" FOOD MARKETING AND TECHNOLOGY, Bd. 8, Nr. 6, Dezember 1994, Seiten 38-40, 42/43, XP002066986

## Beschreibung

Gegenstand der vorliegenden Erfindung ist ein Verfahren zur Herstellung von homogenen Glycerophospholipiden und polaren oder lipophilen Substanzen enthaltenden Formulierungen.

Glycerophospholipide haben eine wichtige physiologische Bedeutung als Bausteine von Membranen, speziell bei der Kompartimentierung in biologischen Systemen. Entsprechend dieser Bedeutung kommen sie ubiquitär in tierischen, pflanzlichen und mikrobiellen Lebensformen vor.

Chemisch gesehen bestehen natürliche Glycerophospholipide aus Glycerin, das in C₁- und C₂-Stellung mit Fettsäuren verestert ist und in C₃-Position einen Phosphatidester trägt. Mengenmäßig am bedeutendsten sind in der Natur mit Abstand die Phosphatidylderivate Phosphatidylcholin, Phosphatidylserin, Phosphatidylethanolamin, Phosphatidylinosit und Phosphatidsäure. Daneben kommen in bestimmten Zellsystemen aber auch andere Phospholipide in höheren Konzentrationen vor, z.B. Plasmalogene, Cardiolipine oder Sphingomyeline.

Prinzipiell besteht die Möglichkeit, Glycerophospholipide mittels chemischer und/oder enzymatischer Syntheseverfahren in vitro aufzubauen, wobei Produkte erhalten werden, deren Struktur den natürlich vorkommenden entsprechen, aber auch "künstlich" sein kann. Heutzutage haben aber bei der industriellen Gewinnung die biologischen Quellen, aufgrund der guten Verfügbarkeit, immer noch Vorrang. Dies gilt insbesondere für Pflanzenlecithine, z.B. aus Sojabohnen, Rapssaat oder Sonnenblumenkernen, die bei der Herstellung von Speiseölen im Raffinationsprozeß als Gemisch verschiedener Phospholipidklassen als sog. "Rohlecithin" anfallen. Als bedeutendste tierische Quelle für Glycerophospholipide ist das Eigelb von Hühnereiern zu nennen, das sich durch einen hohen Gehalt an Phosphatidylcholin auszeichnet. Durch verschiedene Verfahrensweisen, wie z.B. Lösemittelextraktionen, können aus den natürlich vorkommenden Gemischen gezielt bestimmte Klassen von Glycerophospholipiden angereichert oder/und meist unter Zuhilfenahme von chromatographischen Trenntechniken in einheitlicher Form gewonnen werden; auch hier steht das Phosphatidylcholin wieder im Vordergrund.

Durch chemische oder/und enzymatische Verfahren können die natürlich vorkommenden Glycerophospholipide z. B. durch Hydrolyse, Hydroxylierung oder Hydrierung so modifiziert werden, dass sich insbesondere ihre oberflächenaktiven Eigenschaften verändern.

Hauptsächlicher Grund zur technologischen Verwendung von oberflächenaktiven Glycerophospholipiden ist ihre emulgierende Wirkung, die speziell zur Stabilisierung von Emulsionen oder Suspensionen, z.B. traditionell in der Lebensmittelindustrie, in der technischen Industrie oder der Pharmazeutik, genutzt wird. Darüber hinaus können Glycerophospholipide auch physiologisch eingesetzt werden, da sie in vivo wichtige Funktionen, insbesondere als Membranbausteine in biologischen Zellen, haben. Aufgrund dieser Tatsache und ihrer toxikologischen Unbedenklichkeit werden vor allem natürliche Glycerophospholipide, insbesondere Phosphatidylcholine und Kephaline, für Produkte oder Formulierungen eingesetzt, die direkt oder indirekt dem Menschen zugeführt werden können. Interessant ist insbesondere die Verwendung in Lebensmitteln und in pharmazeutischen Produkten. Es ist auch bekannt, dass Arzneimittelwirkstoffe bei Formulierung mit Glycerophospholipiden bezüglich ihrer Resorption, Pharmakokinetik und/oder pharmakologischen Wirkung variiert werden können.

Bei der Herstellung von Emulsionen und Dispersionen, beispielsweise bei der Formulierung pharmazeutischer Wirkstoffe, aber auch in anderen technischen Bereichen, wird oft eine möglichst feine und homogene Verteilung der Wirkstoffe, wie eine kleine Teilchengröße, angestrebt. Formulierungen sowie Verfahren zu deren Herstellung, die es ermöglichen, die bisher erreichte Wirkstoffverteilung über eine Formulierung zu verbessern, sind daher von großem Interesse und können weittragende Bedeutung erlangen.

Zur Formulierung von Glycerophospholipiden mit polaren oder lipophilen Substanzen werden nach dem Stand der Technik insbesondere die drei folgenden Verfahrensweisen herangezogen:
(1) die Formulierung der (gelösten) polaren Substanzen bzw. der lipophilen (flüssigen oder gelösten) Substanzen in membranbildende Glycerophospholipid-Vesikel bzw. Liposomen ("Micellares System"),
(2) die Formulierung über einen Einschluß feiner (mikrokristalliner) Partikel der polaren oder der lipophilen festen Substanz in Glycerophospholipide ("Mikrokristallines System") und
(3) die Formulierung über eine chemische Bindung der polaren oder lipophilen Substanz an Glycerophospholipide ("Chemisches Bindungssystem").

Bei Formulierungen in "micellaren Systemen" werden die polaren Substanzen in polaren Lösemitteln, meist Wasser, gelöst und von einer einfachen oder mehrfachen Membran, z.B. Doppelmembran, bestehend aus oberflächenaktiven Glycerophospholipiden umschlossen. Ein ähnlicher Einschluss vollzieht sich mit den flüssigen oder in geeigneten Lösemitteln gelösten lipophilen Substanzen. Einen aktuellen Überblick über diese Formulierungstechnik liefert H. Hauser, Phospholipid Vesicles in Cevc. G. (ed.), Phospholipid Handbook, Marcel Dekker, New York, 1993, pp. 603-637. Der Aufbau einer micellaren Doppelmembran zur Formulierung polarer Substanzen in Wasser bzw. von lipophilen Substanzen ist jedoch nicht unproblematisch, und es müssen gegebenenfalls bestimmte Phosphorsäureester beispielsweise in Form von Glycerophospholipiden, wie z.B. Phosphatidylcholine, in den Membranen vorhanden sein, um die gewünschte Stabilität zu erzielen. Die Einschlussraten für die zu formulierenden Wirkstoffe sind oft nur gering, so dass bei der Herstellung der Formulierug mit hohen Verlusten an Wirkstoffen gerechnet werden muss. Weiterhin sind oftmals Sterinderivate, z.B. das Cholesterin, für eine membranstabilisierende Wirkung erforderlich. Man hat auch versucht, über eine chemische Bindung ein Addukt zwischen der zur formulierenden Substanz und Cholesterin herzustellen, um dadurch eine Stabilisierung der Membranen zu erreichen (z.B. J. L. Murtha et al., J. Pharm. Sci 83 (9), 1222-8, 1994). Auch wurde versucht, liposomale Formulierungen mittels überkritischem Kohlendioxid herzustellen, wobei auf große Mengen organischer Lösemittel verzichtet werden kann (Frederiksen L. et al, J. Pharmaceutical. Sci. 86, 921-8, 1997). Die Formulierungen von Wirkstoffen bspw. im Zusammenhang mit dem Einsatz der Liposomen-Technik brachte z.B. in der modernen Medizin bei vielen Therapien einen großen Fortschritt, jedoch bestehen beispielsweise bei der parenteralen Anwendung im wesentlichen zwei große Nachteile: Zum einen zeigen Liposomen als künstliche Micellen in vivo nur eine begrenzte Lebenszeit, da insbesondere Lipidaustauschreaktionen an Membranen stattfinden können und dadurch das Membranvesikel Liposom instabilisiert wird oder gar zerfällt, bevor es am eigentlichen Zielort der Therapie angekommen ist. Zum anderen wird insbesondere bei größeren Micellen das mononukleare Phagozytensystem aktiv, was zu unerwünschten immunologischen Nebenreaktionen führt. Aus physikalischen Gründen ist es nicht möglich, die Größe der vesikulären Liposomen beliebig zu verkleinern und so beispielsweise einer Immunantwort zu entziehen, da die Membranoberfläche in Abhängigkeit von ihrer Zusammensetzung ab einer gewissen Micellargröße aufreißt.

Bei Formulierungen von polaren oder lipophilen Substanzen in mikokristalliner Form in Glycerophospholipiden besteht ebenfalls das Problem, dass die Größe der Mikrokristalle nach dem Stand der Technik nicht beliebig verkleinert werden kann. Wendet man z.B. ein Lösemittelsystem an, in dem sowohl die polaren/lipophilen Substanzen als auch die Glycerophospholipide gelöst sind, und unterwirft man diese Lösung z.B. einer Sprühtrocknung, so werden die Löslichkeitsgrenzen der Substanzen und der Glycerophospholipide während der Entfernung des Lösemittels bei unterschiedlichen Lösemittel-Konzentrationen erreicht und man erzielt eine "mikrokristalline Formulierung". Wenn die Zeitdauer für die Entfernung des Lösemittels gegen Null geht, könnte deren Partikelgröße zwar theoretisch weiter verkleinert werden. Dies ist jedoch technisch nicht umsetzbar.

Beispiele für solche mikrokristalline Formulierungen und deren Herstellung sind in EP-A-556392, WO-A-9611669, JP-A-62116516 sowie EP-A-51833 beschrieben. In EP-A-556392 wird die Lyophilisierung einer Fettemulsion zur Herstellung einer wasserfreien Zusammensetzung bestehend aus Glycerophospholipiden, polaren und/oder lipophilen Substanzen und Formulierungshilfsmitteln mit mindestens zwei Hydroxylgruppen offenbart. Bevorzugt verwendet wird als Glycerophospholipid raffiniertes Eilecithin und als Hilfsmittel Glycerin. Auch in WO-A-9611669 wird eine Glycerophospholipidmischung als Lyophilisat offenbart. Das Lyophilisat enthält weiterhin Mannitol als Formulierungshilfsmittel und einen Wirkstoff. In JP-A-62116516 wird die Extraktion von Lecithin aus Sojabohnen und dessen weitere Formulierung beschrieben. In EP-A-51833 wird ein Verfahren zur Herstellung lecithinhaltiger Formulierungen mittels Lösungsmittelextraktion mit anschließender Entfernung des Lösungsmittels offenbart.

In der Patentschrift EP-B-0 493 578 wird versucht, die beschriebene Problematik, dass die Größe der Mikrokristalle nicht beliebig verändert werden kann, durch den Einsatz von überkritischem Kohlendioxid zu umgehen. Jedoch müssen bei dem Verfahren sowohl der Emulgator als auch die zu formulierende Substanz im überkritischem Kohlendioxid löslich sein, um eine entsprechende homogene Verteilung erzielen zu können. Die Löslichkeit von Glycerophospholipiden im überkritischen Kohlendioxid ist aber nur marginal und nur ausgewählte Wirkstoffe können verfahrensgemäß eingesetzt werden. Allein durch die erste Eigenschaft ist das vorgeschlagene Verfahren für Wirkstoff-Formulierungen mit Glycerophospholipiden ungeeignet.

Ein weiteres Verfahren zur Herstellung einer pulverförmigen Arzneiform, die aus einem oder mehreren Wirkstoffen und einem oder mehreren Trägern besteht, und das mit Hilfe eines fluiden Gases durchgeführt wird, ist aus EP-A-0 322 687 bekannt. Dabei wird zunächst ein flüssiges Medium, das die Wirkstoffkomponente und die Trägerkomponente enthält, mit dem fluiden Gas in Kontakt gebracht, dann die Flüssigkeit durch das fluide Gas entfernt und die so vom flüssigen Medium befreite Wirkstoff/Träger-Zubereitung gewonnen. Mit diesem Verfahren, bei dem u.a. CO₂, N₂O und Kohlenwasserstoffe im überkritischen Zustand als fluide Gase und Polymere, Lipide, Lecithin und Wachse als Trägerkomponenten eingesetzt werden, werden über eine sog. Sprüheinbettung pulverförmige Hohlkugeln gewonnen. Die enthaltenen flüssigen Bestandteile der Formulierung werden mit Hilfe eines Sprühturmes und Ein- oder Mehrstoffdüsen vollständig von der pulverförmig anfallenden Zubereitung getrennt. Nachteilig sind bei diesem Verfahren der "Sprüheinbettung" zum einen der technische Aufwand:
(i) Die wertbestimmende Partikelgröße der anfallenden festen Formulierung wird durch eine aufwändige und technisch sehr anfällige Düsenkonstruktion bestimmt.
(ii) Erfahrungsgemäß ist es technisch nur schwer zu realisieren, extrem feine Pulver aus dem Sprühturm zu entnehmen, da die feinen Partikel kaum praktikabel in einem Abscheider gesammelt werden können.

Zum anderen ist es nachteilig, wenn alle flüssigen Bestandteile (also auch die gegebenenfalls vorhandenen flüssigen Formulierungs-Hilfsmittel) vollständig entfernt werden müssen, da das erhaltene mikrokristalline Pulver nur für spezielle Anwendungsformen geeignet ist, wie z. B. zur Herstellung von Mikroverkapselungen in Polymeren mit verzögerter Wirkstoff-Freisetzung.

Bei der Formulierung über eine chemische Bindung einer Substanz mit polarem oder lipophilem Charakter an oberflächenaktive Glycerophospholipide ("Chemisches Bindungssystem"), wie z.B. von Hong et al. in Cancer Res. 50 (14), 4401-6 (1990) beschrieben, besteht der entscheidende Nachteil darin, dass diese Verfahrensweise kompliziert und nicht allgemein anwendbar ist, wobei sich zudem aufgrund der chemischen Bindung die Wirkungsweise der Substanz meist verändert. Die praktische Nutzung dieser Formulierungsstrategie ist somit auf nur wenige Ausnahmen beschränkt.

Die generelle Zielsetzung einer verbesserten Formulierung, nämlich die Herstellung einer molekulardispersen Verteilung zwischen Wirkstoff und Glycerophospholipiden durch eine molekulare, vorzugsweise nichtkovalente Wechselwirkung wird aufgrund der eben beschriebenen Verfahrensweisen nicht erreicht.

Entsprechende Lösungen der o.g. Probleme sind nach dem Stand der Technik nicht bekannt, und Lösungsansätze sind nur für Formulierungen wasserunlöslicher unpolarer Substanzen beschrieben (US-A-4,973,465 oder WO 91/16 068). Es besteht daher ein erhebliches Interesse, die Wirkstoffverteilung insbesondere in pharmazeutischen Formulierungen weiter zu verbessern.

Aufgabe der vorliegenden Erfindung war es deshalb, ein Verfahren zur Herstellung von homogenen wasserfreien Formulierungen aus Wirkstoffen, Trägern und gegebenenfalls Formulierungshilfsmitteln bereitzustellen, mit welchen die beschriebenen Nachteile der bisher bekannten Formulierungen, insbesondere die Ausbildung mikrokristalliner Partikel der zu formulierenden Substanz, zurückgedrängt oder vollständig vermieden werden können.

Gelöst wurde diese Aufgabe durch ein Verfahren zur Herstellung homogener wasserfreier Formulierungen, enthaltend
(A) eine oder mehrere Glycerophospholipide,
(B) eine oder mehrere polare oder/und lipophile Substanzen mit einer Affinität zu Glycerophospholipiden und
(C) gegebenenfalls ein oder mehrere Formulierungshilfsmittel mit mindestens zwei Hydroxylgruppen,
wobei die Glycerophospholipid-Komponente (A) und die Komponente (B) im Molverhältnis von 1:0,001 bis 2 vorliegen und - bei Vorhandensein von (C) - die Glycerophospholipid-Komponente (A) und die Komponente (C) im Molverhältnis von 1:0,001 bis 1 vorliegen, dadurch gekennzeichnet, dass
(a) die Substanz (B) in flüssiger Form bereitgestellt wird, vorzugsweise durch Lösen einem wasserfreien Lösemittel(-gemisch) oder/und dem wasserfreien Formulierungshilfsmittel (C),
(b) Flüssigkeit aus (a) mit einem in einem wasserfreien Lösemittel(gemisch gelösten Glycerophospholipid(-gemisch) (A), das gegebenenfalls das wasserfreie Formulierungshilfsmittel (C) enthält, unter Aufrechterhaltung des gelösten Zustandes vereinigt wird, und
(c) die Lösung aus (b) einer Extraktion zur Entfernung des Lösemittelgemisches unterzogen wird.

Glycerophospholipide im Sinne der vorliegenden Erfindung sind Verbindungen, die einen Glycerinrest enthalten, der mit mindestens einem Fettsäurerest und mit mindestens einem Phosphatidrest verestert ist. Beispiele für geeignete Klassen von Glycerophospholipiden sind Phosphatidsäuren, Phosphatidylester, Lysophospholipide, Cardiolipine und Plasmalogene. Bevorzugt sind Phosphatidylester und Lysophospholipide, die am C₁-Atom des Glycerins mit einem Fettsäurerest und am C₃-Atom des Glycerins mit einem Phosphatidrest verestert sind. Besonders bevorzugt werden die Glycerophospholipide (A) ausgewählt aus Verbindungen der allgemeinen Formel (I): 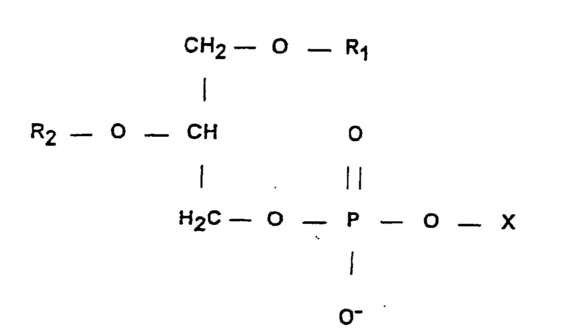 worin R₁ und R₂ gleich oder verschieden sein können und jeweils einen Fettsäureesterrest der allgemeinen Formel (II): 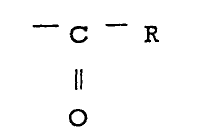 bedeuten, worin R für geradkettige oder verzweigtkettige, gesättigte oder ein- oder mehrfach ungesättigte, gegebenenfalls z.B. mit OH oder Heteroatomen wie 0, N, S in der Kette substituierte C₆- bis C₂₄-Fettsäurereste steht, worin R₂ auch H sein kann und worin X einen Rest aus der Reihe -H, CH₂-CH₂-NH₃⁺, -CH₂-CH₂-N-(CH₃)₃⁺, -CH₂-CH(NH₃⁺)COO⁻, -CH₂-CH(-OH)-CH₂-OH oder 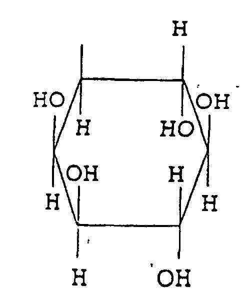 bedeutet.

Als Berechnungsgrundlage für die Molverhältnisse können die mittleren Molgewichte der Glycerophospholipide eingesetzt werden.

Überraschenderweise wurde nun gefunden, dass die durch das erfindungsgemäße Verfahren hergestellten Formulierungen beim Einbringen in hydrophile oder/und lipophile Medien sowie in biphasische Systeme eine hohe Stabilität besitzen und nicht oder nur unwesentlich in die Ausgangskomponenten zerfallen. Die erfindungsgemäßen Formulierungen besitzen ein Lösungsverhalten, das über das Molverhältnis von Glycerophospholipid (A) zur polaren oder lipophilen Substanz (B) gezielt gesteuert werden kann: Bei einem gemäß Erfindung möglichen äquimolaren Verhältnis von Glycerophospholipid zu einer polaren Substanz sind die Formulierungen amphiphil, d.h. sie wirken in einem biphasischen System oberflächenaktiv. Bei einem Überschuß an Glycerophospholipid werden diese Formulierungen mehr lipophil, d.h. sie lassen sich gut in Öl einarbeiten. Beispielsweise bilden so wasserlösliche Farbstoffe, die mit einem Überschuß an Glycerophospholipid formuliert worden sind, in Öl bei Temperaturen >50 °C klare, farbige Lösungen. Durch die zusätzliche Verwendung der Formulierungshilfsmittel (C) kann bei allen durch das erfindungsgemäße Verfahren hergestellten Formulierungen die Dispergierbarkeit oder/und Löslichkeit in Wasser sowie die Stabilität der Emulsion entscheidend verbessert werden. Dies äußert sich beispielsweise bei Formulierungshilfsmittel enthaltenden Formulierungen von unpolaren Wirkstoffen, die bei Temperaturen >50 °C einfach und stabil in Wasser dispergiert werden können, wobei ohne Deaggregation der Formulierung eine Sterilisation über 0,2 µm-Filter durchgeführt werden kann. Bei der Formulierung von lipophilen Substanzen kann die Löslichkeit der Gesamtformulierung in Wasser signifikant verbessert werden.

Besonders gute Eigenschaften im Sinn der Erfindung können erzielt werden, wenn die Glycerophospholipid-Komponente (A) Lecithine oder/und Kephaline enthält.

Als bevorzugte Substanzen (B) mit polaren oder/und lipophilen Eigenschaften werden erfindungsgemäß physiologische Wirkstoffe oder Farbstoffe verwendet, deren Molekulargewicht <1500 Dalton, insbesondere <500 Dalton, betragen sollte.

Ein wesentlicher Aspekt ist, dass die verwendeten Substanzen (B) eine Affinität zu Glycerophospholipiden aufweisen, was nicht zwingend in einer kovalenten chemischen Reaktion resultiert, sondern sich - wie im vorliegenden Fall besonders bevorzugt - durch die Ausbildung von nichtkovalenten Wechselwirkungen wie etwa Nebenvalenzen, sog. Wasserstoffbrückenbindungen oder/und in lipohilen Wechselwirkungen manifestiert. Dabei erfolgt eine Stabilisierung unter Ausbildung der für das erfindungsgemäße Verfahren typischen homogenen, wasserfreien Aggregate ("molecular self assembling"), die von ihrer Struktur her am ehesten mit "erstarrten Lösungen" zu vergleichen sind.

Unter physiologischen Wirkstoffen versteht die vorliegende Erfindung alle Verbindungen oder Stoffklassen, die in Stoffwechselvorgänge regelnd oder steuernd eingreifen. Dabei ist die Wirkung insbesondere bei der Verabreichung an Säuger oder den Menschen selbstverständlich von der Applikationsform abhängig. Vom Ausdruck "polare Substanz" oder "lipophile Substanz" sind demnach alle polaren und z.T. auch wasserlöslichen bzw. lipophilen Arzneimittelwirkstoffe umfasst, die für topische und transdermale Applikationen geeignet sind, oder die peroral, parenteral sowie inhalativ und hier insbesondere intravenös, intramuskulär, subkutan, intraperitonal bzw. intranasal verabreicht werden können.

Miteingeschlossen sind auch Wirkstoffe, die in Kosmetika eingesetzt werden, darüber hinaus aber auch Agrochemikalien, z.B. Düngemittel, Pflanzenwachstumsregulatoren, Herbizide, Insektizide, sowie Biozide allgemeiner Art.

Aus der Vielzahl der möglichen physiologischen Wirkstoffe seien beispielhaft die wasserlöslichen Vitamine oder fettlösliche Vitamine wie z.B. die der Gruppe A, D, E und K angeführt. Ein Vertreter der wasserlöslichen Farbstoffe ist Ponceau 4 R (E 124); wichtige Vertreter der lipophilen Farbstoffe sind die Carotinoide.

Die durch das erfindungsgemäße Verfahren hergestellten Formulierungen sind wasserfrei, d.h. sie enthalten vorzugsweise weniger als 5 Gew.-%, besonders bevorzugt weniger als 3 Gew.-% und am meisten bevorzugt weniger als 1 Gew.-% Wasser bezogen auf das Gesamtgewicht der Formulierung.

Weiterhin sind diese Formulierungen vorzugsweise ölfrei, d.h. sie enthalten vorzugsweise maximal 3 Gew.-% und besonders bevorzugt maximal 2 Gew.-% Öl, d.h. Triglyceride, bezogen auf das Gesamtgewicht der Formulierung. Besonders bevorzugt liegen die durch das erfindungsgemäße Verfahren hergestellten Formulierungen in einer bei Raumtemperatur festen Form, z.B. als rieselfähiges Pulver vor.

Das vorliegende erfindungsgemäße Verfahren sieht ferner vor, dass die hergestellten Formulierungen gegebenenfalls ein oder mehrere Polyolverbindungen als Formulierungshilfsmittel (C) enthalten, worin vorzugsweise flüssige Polyole, insbesondere C₂-bis C₄-Verbindungen verstanden werden, die mindestens zwei Hydroxylgruppen enthalten, ganz besonders bevorzugt Glycerin. Dabei können die in Frage kommenden Polyole auch als beliebige Mischungen eingesetzt werden. Bevorzugt ist aber, dass die Formulierungshilfsmittel zur Herstellung der Formulierung in wasserfreier Form vorliegen, d.h. vorzugsweise mit einem Wassergehalt von maximal 5 Gew.-% bezogen auf das Gewicht des Formulierungshilfsmittels eingesetzt werden.

Das Verfahren der vorliegenden Erfindung wird im Wesentlichen in drei Schritten durchgeführt:

Zunächst wird im Verfahrensschritt (a) die Substanz (B) in flüssiger Form bereitgestellt, z.B. als reine Substanz, wenn sie unter den jeweiligen Bedingungen als Flüssigkeit vorliegt. Vorzugsweise wird die Substanz (B) jedoch einem wasserfreien, d.h. vorzugsweise maximal 5 Gew.-% Wasser enthaltenden, Lösemittel(-gemisch) gelöst, wofür sich vorzugsweise polare oder/und unpolare Lösemittel(-gemische) anbieten. Dem Lösemittel (-gemisch) kann aber auch das wiederum wasserfreie Formulierungshilfsmittel (C) in den genannten Molverhältnissen zugemischt werden. Soll eine polare Substanz formuliert werden, so kann diese auch ausschließlich im wasserfreien Formulierungshilfsmittel gelöst werden.

Als polare Lösemittel sieht die Erfindung solche mit protischem oder/und aprotischem Charakter vor: Als protische Lösemittel haben sich bevorzugt primäre einwertige C₁₋₁₀-Alkohole, sekundäre einwertige C₃₋₁₀-Alkohole und tertiäre einwertige C₄₋₁₀-Alkohole und beliebige Mischungen davon bewährt. Halogenierte C₁₋₁₀-Kohlenwasserstoffe, bevorzugt Chloroform, sowie Ether, wie Diethylether und Tetrahydrofuran (THF), oder auch beliebige Mischungen davon sind bevorzugte Vertreter der aprotischen Lösemittel.

Als unpolare Lösemittel geeignet sind aliphatische oder cyclische C₅₋₁₀-Kohlenwasserstoffe, oder/und Triglyceride.

In speziellen Fällen ist es sinnvoll, wenn im Verfahrensschritt (a) Mischungen aus polarem und unpolarem Lösemittel(-gemisch) im Gewichtsverhältnis von max. 1:1 verwendet werden.

Die aus Verfahrensschritt (a) resultierende Flüssigkeit bzw. Lösung wird anschließend im Verfahrensschritt (b) mit einem Glycerophospholipid (-gemisch) (A) in der Weise vereinigt, dass der gelöste Zustand der Komponenten aufrecht erhalten bleibt. Voraussetzung hierfür ist, dass auch die Glycerophospholipid-Komponente (A) im gelösten Zustand eingesetzt wird, wofür wiederum wasserfreie polare aber auch unpolare Lösemittel oder Gemische daraus besonders geeignet sind. Gegebenenfalls kann zusätzlich das Formulierungshilfsmittel (C) vorhanden sein.

Sollte es zur Erzielung der Lösung nötig sein, unpolare Lösemittel einzusetzen, haben sich in den Verfahrensschritten (a) oder/und (b) wie bereits angedeutet, erfindungsgemäß aliphatische oder cyclische C₅₋₁₀-Kohlenwasserstoffe, bevorzugt Hexan oder/und Cyclohexan, oder/und Triglyceride bewährt. Aus der Gruppe der Triglyceride werden gemäß Erfindung besonders natürlich vorkommende pflanzliche Öle bevorzugt.

Anschließend unterwirft man die aus Verfahrensschritt (b) resultierende Mischung der gelösten Substanzen einer Extraktion zur Entfernung des Lösemittels(-gemisches). Vorzugsweise wird für die Extraktion ein bei Normalbedingungen gasförmige Kohlenwasserstoffe, z.B. Propan oder/und Butan enthaltendes Extraktionsmittel verwendet. Besonders bevorzugt erfolgt die Extraktion bei einem Druck zwischen 1 und 50 MPa und einer Temperatur von 20 bis 150 °C in einer Trennkolonne mit einem Propan oder/und Butan enthaltenden Extraktionsmittel, wobei die Extraktion so geführt wird, dass das Extraktionsgemisch in eine homogene Unterphase aus Glycerophospholipid(-gemisch) (A), Substanz (B) und gegebenenfalls Formulierungs-Hilfsmittel (C) und eine das Lösemittel und gegebenenfalls die Substanz (B) enthaltende Oberphase verteilt wird, sich die Unterphase von der Oberphase trennt und die Formulierung aus der Unterphase, die üblicherweise in schmelzeartiger Form vorliegt, gewonnen wird. Das erfindungsgemäße Verfahren zur Formulierung kann somit ohne wesentliche Verluste an zu formulierender Substanz durchgeführt werden.

Unter erfindungsgemäß bevorzugten Verfahrensbedingungen wird die aus den Verfahrensschritten (a) und (b) stammende Lösung des Ausgangsmaterials in einer Trennkolonne durch ein von unten kommendes Extraktionsmittel(-gemisch) extrahiert, das bevorzugt aus Propan mit bis zu 95 Gew.-% Dimethylether (DME) besteht.

Als Extraktionbedingungen sieht die Erfindung vorzugsweise einen Druck zwischen 1 und 50 MPa sowie eine Temperatur von 20 bis 150 °C vor, wobei sich Druckbereiche zwischen 3 und 20 MPa sowie Temperaturen von 30 bis 100 °C besonders bewährt haben. Nach der Extraktion, die vorzugsweise kontinuierlich in einem Gegenstromprozess durchgeführt wird, wird einerseits das Extraktionsmittel(-gemisch) weggeführt, andererseits nimmt die als Schmelze zum Kolonnensumpf hin absinkende Glycerophospholipid-Fraktion die zu formulierende Substanz (B) gegebenenfalls mit dem Formulierungshilfsmittel auf. Dabei findet die eigentliche Ausbildung der Formulierung zwischen dem Glycerophospholipid(-gemisch), der polaren oder lipophilen Substanz und gegebenenfalls dem Formulierungshilfsmittel statt, was sich vollständig im gelösten Zustand vollziehen kann. Zur besseren Auftrennung des Ausgangsgemisches in die flüssige Oberphase und Unterphase (Schmelze) hat sich die Einstellung eines Temperaturgradienten in der Trennkolonne bestens bewährt, bei dem die Kopftemperatur 5 bis 50 °C über der des Kolonnensumpfes liegt.

Die im Kolonnensumpf vorliegende Schmelze der Formulierung, die üblicherweise einen Gehalt an Extraktionsmittel(-gemisch) zwischen 20 und 40 Gew.-% aufweist, kann über eine geeignete Düsenanordnung auf Umgebungsdruck ausgetragen werden, um sie durch Druckabsenkung oder/und Temperaturerhöhung von Extraktionsmittel(-gemisch) zu befreien. Das als Kopfprodukt anfallende Lösemittel(-gemisch) wird ebenfalls vom Extraktionsmittel(-gemisch) befreit, was zweckmäßig in einem Separator ebenfalls durch Druckabsenkung oder/und Temperaturerhöhung erfolgt. Gegebenenfalls kann das Extraktionsmittel(-gemisch) nach einer Komprimierung recyclisiert werden.

Bei der beschriebenen Herstellungsweise der erfindungsgemäßen Formulierungen ist es besonders bevorzugt, wenn die Schmelze des Glycerophospholipid(-gemisches) (A) die zu formulierende Substanz (B) direkt aus dem Lösemittel(-gemisch) der eingespeisten Mischung übernehmen kann. Dabei treten aufgrund des erfindungsgemäßen Verfahrensablaufes im Gegensatz zu den bislang bekannten Verfahren keine mikrokristallinen Partikel auf. Die beanspruchten Formulierungen liegen deshalb völlig homogen vor.

Ganz allgemein dient das erfindungsgemäße Verfahren also dazu, Substanzen mit polarem oder/und lipophilem Charakter in homogene Formulierungen mit lipophilen oder amphiphilen Eigenschaften zu überführen, so dass sie für bisher nur sehr umständlich oder gar nicht zugängliche Anwendungen nutzbar sind.

Dies ist auch der Grund dafür, dass sich die durch das erfindungsgemäße Verfahren hergestellten homogenen Formulierungen insbesondere zur Bereitung von Dispersionen, Emulsionen und/oder Suspensionen für die Lebensmitteltechnik, die Biotechnologie, die agrochemische, kosmetische und pharmazeutische Industrie, und hier ganz besonders für die Galenik von Wirkstoffen, eignen, aber auch für die Farb-, Lack- und Lederindustrie.

Zur Herstellung von Formulierungen für pharmazeutische Zubereitungen, die gegebenenfalls pharmazeutisch üblichen Träger-, Hilfs-, Füll- oder/und Verdünnungsmitteln enthalten, ist insbesondere auch die Verteilung der in den erfindungsgemäßen stabilen Formulierungen bzw. Aggregaten mit Glycerophospholipiden unterhalb der kritischen Micellkonzentration von Interesse. Diese neuen Möglichkeiten spielen vor allem bei membranalen Penetrationsvorgängen eine wichtige Rolle.

Die nachfolgenden Beispiele veranschaulichen ein typisches Herstellungsverfahren der erfindungsgemäßen Formulierungen:

### Beispiele

### Beispiel 1 Formulierung einer polaren Substanz mit zwei Lösemitteln (Ethanol und Triglyceride) ohne Formulierungshilfsmittel

5,1 g Nicotinsäureamid (polare Substanz) wurden in 84 g Ethanol (99,8 %ig) vollständig gelöst, zu 450 g einer Mischung aus 65 Gew.-% Glycerophospholipiden (natürliches Gemisch aus Sojabohnen) und 35 Gew.-% Triglyceriden (Sojaöl) gegeben und sorgfältig durch Rühren bei 45 °C durchmischt. Die so erhaltene Mischung wurde mittels einer Hochdruckpumpe einer Extraktionskolonne etwa in der Mitte zugeführt. Der Verstärkerteil der Kolonne besitzt etwa 5, der Abtriebsteil etwa 7 theoretische Trennstufen. Als Extraktionsmittel diente verdichtetes Propan bei einem Druck von 60 bar. Am Zulauf der Kolonne betrug die Temperatur ca. 75 °C, im Kolonnenkopf 85 °C und im Kolonnensumpf 65 °C. Das Verhältnis von zugepumpter Mischung (Feed) zum Extraktionsmittel Propan betrug durchschnittlich 5 Gew.-%. Die Leerrohr-Geschwindigkeit des Extraktionsmittels in der Kolonne betrug 2 mm/s.

Das über den Kolonnenkopf abgeführte, mit Öl und Ethanol beladene Extraktionsmittel wurde in einem Abscheider bei ca. 70 °C und 8 bar verdampft, wobei das Öl und weitgehend das Ethanol abgeschieden wurden. Die verfahrensgemäß gebildete ölfreie Formulierung (Ölanteil kleiner 2 Gew.-%) wurde am Sumpf der Extraktionskolonne über eine Düsenanordnung auf Normaldruck ausgetragen, wobei durch die spontane Verdampfung des Propans eine Abkühlung der Formulierung stattfand und ein gut rieselfähiges Pulver gewonnen wurde. Der Anteil an Nicotinsäureamid in der homogenen Formulierung betrug 1,7 Gew.-%, was einem molaren Verhältnis von 0,1 entspricht (berechnet auf Basis eines durchschnittlichen Molekulargewichts der Glycerophospholipide von 700 Dalton).

Eine chemische Analyse der Einzelkomponenten der Formulierung bestätigte die konstante Zusammensetzung während des Extraktionsverlaufes.

### Beispiel 2 Formulierung einer polaren Substanz mit einem Formulierungshilfsmittel und zwei Lösemitteln (Triglyceride und Ethanol)

16 g Nicotinsäureamid (polare Substanz) wurden in einem Gemisch aus 30 g Ethanol (99,8 %ig) und 21 g Glycerin (wasserfreies Formulierungshilfsmittel) bei 45 °C vollständig gelöst, zu 500 g einer Mischung aus 65 Gew.-% Glycerophospholipiden (natürliches Gemisch aus Sojabohnen) und 35 Gew.-% Triglyceriden (Sojaöl) gegeben und sorgfältig durch Rühren wiederum bei 45 °C durchmischt. Die Mischung der gelösten Substanzen wurde mittels einer Hochdruckpumpe einer Extraktionskolonne etwa in der Mitte zugeführt. Der Verstärkerteil der Kolonne besitzt etwa 5, der Abtriebsteil etwa 7 theoretische Trennstufen. Als Extraktionsmittel diente verdichtetes Propan bei einem Druck von 50 bar. Am Zulauf der Kolonne betrug die Verfahrenstemperatur ca. 70 °C, im Kolonnenkopf 80 °C und im Kolonnensumpf 60 °C. Das Verhältnis von zugepumpter Mischung (Feed) zum Extraktionsmittel Propan betrug durchschnittlich 3 Gew.-%. Die Leerohr-Geschwindigkeit des Extraktionsmittels in der Kolonne betrug 2 mm/s.

Das über den Kolonnenkopf abgeführte, mit Öl und Ethanol beladene Extraktionsmittel wurde in einem Abscheider bei ca. 70 °C und 8 bar verdampft, wobei das Öl und weitgehend das Ethanol abgeschieden wurde. Die verfahrensgemäß gebildete ölfreie Formulierung (Ölanteil kleiner 2 Gew.-%) wurde am Sumpf der Extraktionskolonne über eine Düsenanordnung auf Normaldruck ausgetragen, wobei durch die spontane Verdampfung des Propans eine Abkühlung der Formulierung stattfand und ein rieselfähiges Pulver gewonnen wurde. Der Anteil an Nicotinsäureamid in der Formulierung betrug 4,4 Gew.-%, was einem molaren Verhältnis von 0,28 bezogen auf das Glycerophospholipid entspricht; der Anteil von 5,8 Gew.-% Glycerin in der Formulierung entspricht einem molaren Verhältnis von 0,5 bezogen auf das Glycerophospholipid (berechnet auf Basis eines durchschnittlichen Molekulargewichts der Glycerophospholipide von 700 Dalton).

### Beispiel 3 Formulierung einer polaren Substanz mit einem Formulierungshilfsmittel und einem Lösemittel (Triglyceride)

0,9 g Nicotinsäureamid (polare Substanz) wurden in 15 g Glycerin (wasserfreies Formulierungshilfsmittel) bei 45 °C vollständig gelöst, zu 540 g einer Mischung bestehend aus 60 Gew.-% Glycerophospholipiden (natürliches Gemisch aus Sojabohnen) und 40 Gew.-% Triglyceriden (Sojaöl) gegeben und sorgfältig durch Rühren wiederum bei 45 °C durchmischt. Die Mischung der gelösten Substanzen wurde mittels einer Hochdruckpumpe einer Extraktionskolonne etwa in der Mitte zugeführt. Der Verstärkerteil der Kolonne besitzt etwa 5, der Abtriebsteil etwa 7 theoretische Trennstufen. Als Extraktionsmittel diente verdichtetes Propan mit einem Butananteil von 25 Gew.-% bei einem Druck von 60 bar. Am Zulauf der Kolonne betrug die Verfahrenstemperatur ca. 75 °C, im Kolonnenkopf 85 °C und im Kolonnensumpf 65 °C. Das Verhältnis von zugepumpter Mischung (Feed) zum Extraktionsmittelgemisch Propan/Butan betrug durchschnittlich 5 Gew.-%. Die Leerrohrgeschwindigkeit des Extraktionsmittelgemisches in der Kolonne betrug 2 mm/s.

Das über den Kolonnenkopf abgeführte, mit Öl beladene (aber Glycerinund Nicotinsäureamid-freie) Extraktionsmittelgemisch wurde in einem Abscheider bei ca. 70 °C und 6 bar verdampft, wobei das Öl abgeschieden wurde. Die verfahrensgemäß gebildete ölfreie Formulierung (Ölanteil kleiner 2 Gew.-%) wurde am Sumpf der Extraktionskolonne über eine Düsenanordnung auf Normaldruck ausgetragen, wobei durch die spontane Verdampfung des Extraktionsmittelgemisches eine Abkühlung der Formulierung stattfand und ein rieselfähiges Pulver gewonnen wurde. Der Anteil an Nicotinsäureamid in der Formulierung betrug 0,3 Gew.-%, was einem molaren Verhältnis von 0,02 bezogen auf das Glycerophospholipid entspricht; der Anteil an Glycerin in der Formulierung betrug 4,4 Gew.-%, was einem molaren Verhältnis von 0,35 bezogen auf das Glycerophospholipid entspricht (berechnet auf Basis eines durchschnittlichen Molekulargewichts der Glycerophospholipide von 700 Dalton).

### Beispiel 4 Formulierung einer lipophilen Substanz mit zwei Lösemitteln (Ethanol und Triglyceride)

38 g DL-α-Tocopherol (lipophile Substanz) wurden zu 231 g eines gelösten Gemisches bestehend aus 56 Gew.-% Glycerophospholipiden (natürliches Gemisch aus Sojabohnen), 30 Gew.-% Triglyceriden (Sojaöl), 5 Gew.-% wasserfreies Glycerin (Formulierungshilfsmittel) und 9 Gew.-% Ethanol (99,8%ig) gegeben und sorgfältig durch Rühren bei 45 °C durchmischt. Die Mischung der gelösten Substanzen wurde mittels einer Hochdruckpumpe einer Extraktionskolonne etwa in der Mitte zugeführt. Der Verstärkerteil der Kolonne besitzt etwa 6, der Abtriebsteil etwa 6 theoretische Trennstufen. Als Extraktionsmittel diente verdichtetes Propan bei einem Druck von 40 bar. Am Zulauf der Kolonne betrug die Temperatur ca. 70 °C, im Kolonnenkopf 75 °C und im Kolonnensumpf 65 °C. Das Verhältnis von zugepumpter Mischung (Feed) zum Extraktionsmittel Propan betrug durchschnittlich 6 Gew.-%. Die Leerrohr-Geschwindigkeit des Extraktionsmittels in der Kolonne betrug 2 mm/s.

Das über den Kolonnenkopf abgeführte, mit Öl, Ethanol und Anteilen von Tocopherol beladene Extraktionsmittel wurde in einem Abscheider bei ca. 70 °C und 8 bar verdampft, wobei das schwerflüchtige Öl, die Tocopherolanteile und weitgehend das Ethanol abgeschieden wurden. Die ölfreie Formulierung (Ölanteil < 2 Gew.-%) wurde am Sumpf der Extraktionskolonne über eine Düsenanordnung auf Normaldruck ausgetragen, wobei durch die spontane Verdampfung des Propans eine Abkühlung der Formulierung stattfand und ein gut rieselfähiges Pulver erhalten wurde. Der Anteil an DL-α-Tocopherol in der Formulierung beträgt ca. 6 Gew.-%, was einem molaren Verhältnis von 0,1 entspricht. Der Anteil an Glycerin in der Formulierung betrug ca. 7 Gew.-%, was einem molaren Verhältnis von 0,6 entspricht (berechnet auf Basis eines durchschnittlichen Molekulargewichts des Glycerophospholipids von 700 Dalton).

Die hergestellte Formulierung zeigt eine hervorragende Dispergierbarkeit und Stabilität in Wasser.

### Beispiel 5 Formulierung einer polaren wasserlöslichen Substanz mit einem Formulierungshilfsmittel und einem Lösemittel (Triglyceride)

215 mg des Farbstoffes Ponceau 4R (E-124) wurden in 8 g wasserfreiem Glycerin bei 50 °C vollständig gelöst, zu 400 g eines Gemisches bestehend aus 65 Gew.-% Glycerophospholipiden (natürliches Gemisch aus Sojabohnen) und 35 Gew.-% Triglyceriden (Sojaöl) gegeben und sorgfältig durch Rühren bei 50 °C durchmischt. Die Mischung der gelösten Substanzen wurde mittels einer Hochdruckpumpe einer Extraktionskolonne etwa in der Mitte zugeführt. Der Verstärkerteil der Kolonne hatte etwa 5, der Abtriebs-teil etwa 7 theoretische Trennstufen. Als Extraktionsmittel diente verdichtetes Propan bei einem Druck von 60 bar. Am Zulauf der Kolonne betrug die Verfahrenstemperatur ca. 75 °C, im Kolonnenkopf 85 °C und im Kolonnensumpf 65 °C. Das Verhältnis von zugepumpter Mischung (Feed) zum Extraktionsmittel (Propan) war durchschnittlich 5 Gew.-%. Die Leerrohrgeschwindigkeit des Extraktionsmittels in der Kolonne betrug 2 mm/s.

Das über den Kolonnenkopf abgeführte, mit Öl beladene Extraktionsmittel wurde in einem Abscheider bei ca. 70 °C und 8 bar verdampft, wobei das farblöse Öl abgeschieden wurde. Die gebildete ölfreie Formulierung (Ölanteil kleiner 2 Gew.-%) wurde am Sumpf der Extraktionskolonne über eine Düsenanordnung auf Normaldruck ausgetragen, wobei durch die spontane Verdampfung des Propans eine Abkühlung der Formulierung stattfand und ein gut rieselfähiges, rot gefärbtes Pulver gewonnen wurde.

Der Farbstoffanteil in der Formulierung betrug ca. 0,08 Gew.-%, was einem molaren Verhältnis von ca. 0,001 entspricht; der Anteil an Glycerin in der Formulierung betrug ca. 3 Gew.-%, was einem molaren Verhältnis von ca. 0,23 jeweils bezogen auf das Glycerophospholipid entspricht (berechnet mit einem durchschnittlichen Molekulargewicht der Phospholipide von 700 Dalton).

Die so hergestellte Formulierung des Farbstoffes wurde bei einer Temperatur von 50 °C 2%ig in gebleichtes Sojaöl eingerührt, wodurch eine klare, rotgefärbte Öllösung erhalten wurde, die auch nach Abkühlen auf Raumtemperatur stabil war. Bei entsprechenden Vergleichsversuchen mit nichtformuliertem Farbstoff und Lecithin konnte keine analoge Anfärbung des Öls mit dem wasserlöslichen Farbstoff erzielt werden.

### Beispiel 6 Formulierung einer polaren wasserlöslichen Substanz mit zwei Lösemitteln (Triglyceride und Ethanol) ohne Formulierungshilfsmittel

17,2 g Glycolsäure wurden in 25 g Ethanol (96%-ig) bei Raumtemperatur vollständig gelöst, zu 300 g eines Gemisches bestehend aus 64 Gew.-% Glycerophospholipiden (natürliches Gemisch aus Sojabohnen) und 36 Gew.-% Triglyceriden (Sojaöl) gegeben und sorgfältig durch Rühren bei 45 °C durchmischt. Die Mischung der gelösten Substanzen wurde mittels einer Hochdruckpumpe einer Extraktionskolonne etwa in der Mitte zugeführt. Der Verstärkeranteil der Kolonne besaß etwa 5, der Abtriebsteil etwa 7 theoretische Trennstufen. Als Extraktionsmittel diente verdichtetes Propan bei einem Druck von 45 bar. Am Zulauf der Kolonne betrug die Verfahrenstemperatur ca. 75 °C, im Kolonnenkopf 85 °C und im Kolonnensumpf 65 °C. Das Verhältnis von zugepumpter Mischung (Feed) zum Extraktionsmittel (Propan) war durchschnittlich 5 Gew.-%. Die Leerrohrgeschwindigkeit des Extraktionsmittels in der Kolonne war 2 mm/s.

Das über den Kolonnensumpf abgeführte, mit Öl und Ethanol beladene Extraktionsmittel wurde in einem Abscheider bei ca. 70°C und 8 bar verdampft, wobei das Öl und das Ethanol abgeschieden wurden. Die gebildete ölfreie Formulierung (Öl- und Ethanolanteil kleiner 2 Gew.-%) wurde am Sumpf der Extraktionskolonne über eine Düsenanordnung auf Normaldruck ausgetragen, wobei durch die spontane Verdampfung des Propans eine Abkühlung der Formulierung stattfand und ein gut rieselfähiges Pulver gewonnen werden konnte. Der Anteil an Glykolsäure in der Formulierung betrug ca. 8 Gew.-%, was einem molaren Verhältnis von ca. 0,8 entspricht (berechnet auf Basis eines durchschnittlichen Molekulargewichts der Phospholipide von 700 Dalton).

Die so hergestellte Formulierung der Glykolsäure wurde unter leichtem Erwärmen 50%ig in n-Hexan gelöst, wodurch eine klare, stabile Lösung erhalten wurde.

### Beispiel 7 Formulierung einer polaren wasserlöslichen Substanz mit Formulierungshilfsmittel und zwei Lösemitteln (Triglyceride und Methanol)

6,5 g Ascorbinsäure wurden in einem Gemisch aus 19,1 g Methanol und 5,8 g wasserfreiem Glycerin bei 45 °C vollständig gelöst zu 300 g eines Gemisches bestehend aus 64 Gew.-% Glycerophospholipiden (natürliches Gemisch aus Sojabohnen) und 36 Gew.-% Triglyceriden (Sojaöl) gegeben und sorgfältig durch Rühren bei 60 °C durchmischt. Die Mischung der gelösten Substanzen wurde mittels einer Hochdruckpumpe einer Extraktionskolonne etwa in der Mitte zugeführt. Der Verstärkerteil der Kolonne besaß etwa 5, der Abtriebsteil etwa 7 theoretische Trennstufen. Als Extraktionsmittel diente verdichtetes Propan bei einem Druck von 50 bar. Am Zulauf der Kolonne betrug die Verfahrenstemperatur ca. 75 °C, im Kolonnenkopf 85 °C und im Kolonnensumpf 65 °C. Das Verhältnis von zugepumpter Mischung (Feed) zum Extraktionsmittel (Propan) war durchschnittlich 5 Gew.-%. Die Leerrohrgeschwindigkeit des Extraktionsmittels in der Kolonne war 2 mm/s.

Das über den Kolonnenkopf abgeführte, mit Öl und Methanol beladene Extraktionsmittel wurde in einem Abscheider bei ca. 70 °C und 8 bar verdampft, wobei das Öl und das Methanol abgeschieden wurden. Die gebildete ölfreie Formulierung (Öl- und Methanolanteil kleiner 2 Gew.-%) wurde am Sumpf der Extraktionskolonne über eine Düsenanordnung auf Normaldruck ausgetragen, wobei durch die spontane Verdampfung des Propans eine Abkühlung der Formulierung stattfand und ein gut rieselfähiges Pulver gewonnen werden konnte. Der Anteil an Ascorbinsäure in der Formulierung betrug ca. 3,5 Gew.-%, der Anteil an Glycerin ca. 3 Gew.-%, dies entspricht einem molaren Verhältnis von 0,13 bzw. 0,23 (berechnet auf Basis eines durchschnittlichen Molekulargewichts der Phospholipide von 700 Dalton).

Die Formulierung wurde 1 %ig in Schweineschmalz gelöst und einem beschleunigten Oxidationstest bei 110°C in einem sog. Rancimaten unterzogen. Die Formulierung zeigte eine signifikante Verbesserung der Oxidationsstabilität im Vergleich zum Blindwert (formulierte Matrix aus Lecithin und Glycerin ohne Ascorbinsäure).

### Beispiel 8 Formulierung einer polaren Substanz mit Formulerungshilfsmittel und zwei Lösemitteln (Triglyceride und Ethanol)

1,8 g Ketoprofen wurden in einem Gemisch aus 10 g Ethanol und 5 g wasserfreiem Glycerin bei 45 °C vollständig gelöst, zu 156 g eines Gemisches bestehend aus 64 Gew.-% Glycerophospholipiden (natürliches Gemisch aus Sojabohnen) und 36 Gew.-% Triglyceriden (Sojaöl) gegeben und sorgfältig durch Rühren bei 60 °C durchmischt. Die Mischung der gelösten Substanzen wurde mittels einer Hochdruckpumpe einer Extraktionskolonne etwa in der Mitte zugeführt. Der Verstärkerteil der Kolonne besaß etwa 5, der Abtriebsteil etwa 7 theoretische Trennstufen. Als Extraktionmittel diente verdichtetes Propan bei einem Druck von 50 bar. Am Zulauf der Kolonne betrug die Verfahrenstemperatur ca. 75 °C, im Kolonnenkopf 85 °C und im Kolonnensumpf 65 °C. Das Verhältnis von zugepumpter Mischung (Feed) zum Extraktionsmittel (Propan) war durchschnittlich 4 Gew.-%. Die Leerrohrgeschwindigkeit des Extraktionsmittels in der Kolonne war 2 mm/s.

Das über den Kolonnenkopf abgeführte, mit Öl und Ethanol beladene Extraktionsmittels wurde in einem Abscheider bei ca. 70 °C und 8 bar verdampft, wobei das Öl und das Ethanol abgeschieden wurden. Die gebildete ölfreie Formulierung (Öl- und Ethanolanteil kleiner 2 Gew.-%) wurde am Sumpf der Extraktionskolonne über eine Düsenanordnung auf Normaldruck ausgetragen, wobei durch die spontane Verdampfung des Propans eine Abkühlung der Formulierung stattfand und ein gut rieselfähiges Pulver gewonnen werden konnte. Der Anteil an Ketoprofen in der Formulierung betrugt ca. 11 Gew.-%, der Anteil an Glycerin ca. 5 Gew.-%; dies entspricht einem molaren Verhältnis von 0,32 bzw. 0,38 (berechnet auf Basis eines durchschnittlichen Molekulargewichts der Phospholipide von 700 Dalton).

Die pulverförmige Formulierung zeigte eine hervorragende Dispergierbarkeit in wässrigen Medien.

### Beispiel 9 Formulierung einer polaren Substanz in lyso-Lecithin mit zwei Lösemitteln (Triglyceride und Ethanol) ohne Formulierungshilfsmittel

10 g Salicylsäure wurden in 10 g Ethanol bei Raumtemperatur vollständig gelöst, zu 192 g eines Gemisches bestehend aus 64 Gew-% enzymatisch hydrolysierten Glycerophospholipiden (Emulfluid E, Lucas Meyer, natürliches Gemisch aus Sojabohnen) und 36 Gew.-% Triglyceriden (Sojaöl) gegeben und dieses Gemenge sorgfältig durch Rühren bei 60 °C durchmischt. Die Mischung der gelösten Substanzen wurde mittels einer Hochdruckpumpe einer Extraktionskolonne etwa in der Mitte zugeführt. Der Verstärkerteil der Kolonne besaß etwa 5, der Abtriebsteil etwa 7 theoretische Trennstufen. Als Extraktionsmittel diente verdichtetes Propan bei einem Druck von 70 bar. Am Zulauf der Kolonne betrug die Verfahrenstemperatur ca. 75 °C, im Kolonnenkopf 95 °C und im Kolonnensumpf 55 °C. Das Verhältnis von zugepumpter Mischung (Feed) zum Extraktionsmittel (Propan) war durchschnittlich 3 Gew.-%. Die Leerrohrgeschwindigkeit des Extraktionsmittels in der Kolonne war 2 mm/s.

Das über den Kolonnenkopf abgeführte, mit Öl und Ethanol beladene Extraktionsmittel wurde in einem Abscheider bei ca. 70 °C und 8 bar verdampft, wobei das Öl und das Ethanol abgeschieden wurden. Die gebildete ölfreie Formulierung (Öl- und Ethanolanteil kleiner 2 Gew.-%) wurde am Sumpf der Extraktionskolonne über eine Düsenanordnung auf Normaldruck ausgetragen, wobei durch die spontane Verdampfung des Propans eine Abkühlung der Formulierung stattfand und ein gut rieselfähiges, feinteiliges Pulver gewonnen werden konnte. Der Anteil an Salicylsäure in der Formulierung betrug ca. 5 Gew.-%, dies entspricht einem molaren Verhältnis von 0,18 (berechnet auf Basis eines durchschnittlichen Molekulargewichts der hydrolysierten Phospholipide von 500 Dalton).

Die pulverförmige Formulierung zeigte eine hervorragende Dispergierbarkeit in wässrigen Medien.

## Patentansprüche

1. Verfahren zur Herstellung von homogenen wasserfreien Formulierungen, enthaltend
(A) ein oder mehrere Glycerophospholipide,
(B) eine oder mehrere polare oder/und lipophile Substanzen mit einer Affinität zu Glycerophospholipiden und
(C) gegebenenfalls ein oder mehrere Formulierungshilfsmittel mit mindestens zwei Hydroxylgruppen,
wobei die Glycerophospholipid-Komponente (A) und die Komponente (B) im Molverhältnis von 1:0,001 bis 2 vorliegen und - bei Vorhandensein von (C) - die Glycerophospholipid-Komponente (A) und die Komponente (C) im Molverhältnis von 1:0,001 bis 1 vorliegen,
**dadurch gekennzeichnet,**
**dass**
(a) die Substanz (B) in flüssiger Form bereitgestellt wird, vorzugsweise durch Lösen in einem wasserfreien Lösemittel(-gemisch) oder/und dem wasserfreien Formulierungshilfsmittel (C),
(b) Flüssigkeit aus (a) mit einem in einem wasserfreien Lösemittel(-gemisch) gelösten Glycerophospholipid(-gemisch) (A), das gegebenenfalls das wasserfreie Formulierungshilfsmittel (C) enthält, unter Aufrechterhaltung des gelösten Zustandes vereinigt wird, und
(c) die Lösung aus (b) einer Extraktion zur Entfernung des Lösemittelgemisches unterzogen wird.

2. Verfahren nach Anspruch 1,
**dadurch gekennzeichnet,**
**dass** die Lösung aus (b) mit einem Propan oder/und Butan enthaltende Extraktionsmittel in einer Trennkolonne bei einem Druck zwischen 1 und 50 MPa und einer Temperatur von 20 bis 150 °C extrahiert wird, wobei das Extraktionsgemisch in eine homogene Unterphase aus Glycerophospholipid(-gemisch) (A), Substanz (B) und gegebenenfalls Formulierungshilfsmittel (C) und eine das Lösemittel und gegebenenfalls die Substanz (B) enthaltende Oberphase separiert, gegebenenfalls die Substanz (B) durch Überschreiten der Löslichkeitsgrenze von der Oberphase in die Unterphase verteilt wird, sich die Unterphase von der Oberphase trennt und die Formulierung aus der Unterphase gewonnen wird.

3. Verfahren nach Anspruch 1 oder 2,
**dadurch gekennzeichnet,**
**dass** in den Verfahrensschritten (a) oder/und (b) ein Lösemittel (-gemisch) enthaltend aliphatische oder cyclische C₅₋₁₀-Kohlenwasserstoffe, bevorzugt Hexan oder/und Cyclohexan, oder/und Triglyceride, bevorzugt natürlich vorkommende pflanzliche Öle, eingesetzt wird.

4. Verfahren nach einem der Ansprüche 1 bis 3,
**dadurch gekennzeichnet,**
**dass** in den Verfahrensschritten (a) oder/und (b) ein Lösemittel (-gemisch) enthaltend polare protische oder/und aprotische Lösemittel eingesetzt wird.

5. Verfahren nach Anspruch 4,
**dadurch gekennzeichnet,**
**dass** ein protisches Lösemittel ausgewählt aus primären einwertigen C₁₋₁₀-Alkoholen, sekundären einwertigen C₃₋₁₀-Alkoholen, tertiären einwertigen C₄₋₁₀-Alkoholen und Mischungen davon eingesetzt wird.

6. Verfahren nach Anspruch 4,
**dadurch gekennzeichnet,**
**dass** ein aprotisches Lösemittel ausgewählt aus halogenierten C₁₋₁₀-Kohlenwasserstoffen, bevorzugt Chloroform, Ethern, bevorzugt Diethylether und Tetrahydrofuran (THF); und Mischungen davon eingesetzt wird.

7. Verfahren nach einem der Ansprüche 1 bis 6,
**dadurch gekennzeichnet,**
**dass** im Verfahrensschritt (c) als Extraktionsmittel Propan mit bis zu 95 Gew.-% Dimethylether (DME) eingesetzt wird.

8. Verfahren nach einem der Ansprüche 1 bis 7,
**dadurch gekennzeichnet,**
**dass** die Extraktion im Verfahrensschritt (c) bei einem Druck zwischen 3 bis 20 MPa sowie einer Temperatur von 30 bis 100 °C durchgeführt wird.

9. Verfahren nach einem der Ansprüche 1 bis 8,
**dadurch gekennzeichnet,**
**dass** die Extraktion kontinuierlich in einem Gegenstrom-Prozess durchgeführt wird.

10. Verfahren nach einem der Ansprüche 2 bis 8,
**dadurch gekennzeichnet,**
**dass** in der Trennkolonne ein Temperaturgradient in der Art eingestellt wird, dass die Kopftemperatur 5 bis 50 °C über der des Kolonnensumpfes liegt.

11. Verfahren nach einem der Ansprüche 2 bis 10,
**dadurch gekennzeichnet,**
**dass** die die Formulierung enthaltende Unterphase durch Druckabsenkung oder/und Temperaturerhöhung vom Extraktionsmittel befreit wird.

12. Verfahren nach einem der Ansprüche 1 bis 11,
**dadurch gekennzeichnet,**
**dass** man die Formulierung als rieselfähiges Pulver gewinnt.

## Claims

1. Method for the production of homogeneous, anhydrous formulations containing
(A) one or more glycerophospholipids,
(B) one or more polar and/or lipophilic substances showing an affinity with glycerophospholipids and
(C) maybe one or more formulation aids with at least two hydroxyl groups,
the glycerophospholipid component (A) and the component (B) being present in a molar ratio of 1:0.001 to 2 and - in cases where (C) is present- the glycerophospholipid component (A) and the component (C) being present in a molar ratio of 1:0.001 to 1,
**characterized in that**
(a) substance (B) is provided in liquid form, preferably by means of dissolution in an anhydrous solvent (mixture) and/or in the anhydrous formulation aid (C),
(b) the liquid from (a) is combined with a glycerophospholipid (mixture) (A) dissolved in an anhydrous solvent (mixture) and maybe containing an anhydrous formulation aid (C), the dissolved state of the components being maintained, and
(c) the solution from (b) is subjected to an extraction process to remove the solvent mixture.

2. Method according to claim 1,
**characterized in that**
the solution from (b) is extracted in a rectifying column with an extracting agent containing propane and/or butane under a pressure between 1 and 50 MPa and a temperature from 20 to 150 °C, the extraction mixture separating into a homogeneous lower phase comprising glycerophospholipid (mixture) (A), substance (B) and maybe formulation aid (C), and an upper phase containing the solvent and maybe substance (B), with substance (B) maybe being distributed from the upper phase to the lower phase due to its exceeding the solubility limit, the lower phase separating from the upper phase, and the formulation being obtained from the lower phase.

3. Method according to claim 1 or 2,
**characterized in that**
in steps (a) and/or (b), use is made of a solvent (mixture) containing aliphatic or cyclic C₅₋₁₀ hydrocarbons, preferably hexane and/or cyclohexane, and/or triglycerides, in the latter case preference being given to natural vegetable oils.

4. Method according to one of claims 1 to 3,
**characterized in that**
in steps (a) and/or (b), use is made of a solvent (mixture) containing polar protic and/or aprotic solvents.

5. Method according to claim 4,
**characterized in that**
use is made of a protic solvent selected from the primary monovalent C₁₋₁₀ alcohols, secondary monovalent C₃₋₁₀ alcohols and tertiary monovalent C₄₋₁₀ alcohols, as well as mixtures thereof.

6. Method according to claim 4,
**characterized in that**
use is made of an aprotic solvent selected from halogenated C₁₋₁₀ hydrocarbons, preferably chloroform, ethers, preferably diethyl ether, and tetrahydrofuran (THF), and also mixtures thereof.

7. Method according to one of claims 1 to 6,
**characterized in that**
in step (c), propane with up to 95 wt. % dimethyl ether (DME) is used as extracting agent.

8. Method according to one of claims 1 to 7,
**characterized in that**
the extraction in step (c) is carried out under a pressure between 3 and 20 MPa and at a temperature from 30 to 100 °C.

9. Method according to one of claims 1 to 8,
**characterized in that**
the extraction is carried out as a continuous countercurrent process.

10. Method according to one of claims 2 to 8,
**characterized in that**
a temperature gradient is set in the rectifying column such that the temperature at the top of the column is 5 to 50 °C higher than that at the bottom of the column.

11. Method according to one of claims 2 to 10,
**characterized in that**
the lower phase containing the formulation is freed of extracting agent by way of a pressure reduction and/or by raising the temperature.

12. Method according to one of claims 1 to 11,
**characterized in that**
the formulation is obtained as a free-flowing powder.

## Revendications

1. Procédé de préparation de formulations anhydres homogènes, contenant
(A) un ou plusieurs glycérophospholipides,
(B) une ou plusieurs substances polaires et/ou lipophiles ayant une affinité pour les glycérophospholipides, et
(C) éventuellement un ou plusieurs auxiliaires de formulation ayant au moins deux groupes hydroxyle,
le composant glycérophospholipidique (A) et le composant (B) se trouvant en un rapport molaire de 1:0,001 à 2 et - en cas de présence de (C) - le composant glycérophospholipidique (A) et le composant (C) se trouvant en un rapport molaire de 1:0,001 à 1,
**caractérisé en ce que**
(a) on prépare la substance (B) sous forme liquide, de préférence par dissolution dans un solvant (ou un mélange de solvants) anhydre et/ou dans l'auxiliaire de formulation (C) anhydre,
(b) on mélange le liquide de (a) avec un glycérophospholipide (ou un mélange de glycérophospholipides) (A) dissous dans un solvant (ou un mélange de solvants) anhydre, contenant éventuellement l'auxiliaire de formulation (C), en maintenant l'état dissous, et
(c) on soumet la solution de (b) à une extraction pour éliminer le mélange de solvants.

2. Procédé selon la revendication 1, **caractérisé en ce que** l'on extrait la solution de (b) avec un agent d'extraction contenant du propane et/ou du butane dans une colonne de séparation sous une pression comprise entre 1 et 50 MPa et à une température de 20 à 150°C, grâce à quoi le mélange d'extraction se sépare en une phase inférieure homogène de glycérophospholipide (ou du mélange de glycérophospholipides) (A), de la substance (B) et éventuellement de l'auxiliaire de formulation (C) et une phase supérieure contenant le solvant et éventuellement la substance (B), on fait passer éventuellement la substance (B) de la phase supérieure dans la phase inférieure par dépassement de la limite de solubilité, on sépare la phase inférieure de la phase supérieure et on récupère la formulation à partir de la phase inférieure.

3. Procédé selon la revendication 1 ou 2, **caractérisé en ce que**, dans les étapes de procédé (a) et/ou (b), on utilise un solvant (ou un mélange de solvants) contenant des hydrocarbures aliphatiques ou cycliques en C₅-C₁₀, de préférence de l'hexane et/ou du cyclohexane, et/ou des triglycérides, de préférence des huiles végétales naturelles.

4. Procédé selon l'une des revendications 1 à 3, **caractérisé en ce que**, dans les étapes de procédé (a) et/ou (b), on utilise un solvant (ou un mélange de solvants) contenant des solvants polaires protiques et/ou aprotiques.

5. Procédé selon la revendication 4, **caractérisé en ce que** l'on utilise un solvant protique choisi parmi des oalcools primaires monovalents en C₁-C₁₀, des alcools secondaires monovalents en C₃-C₁₀, des alcools tertiaires monovalents en C₄-C₁₀ et leurs mélanges.

6. Procédé selon la revendication 4, **caractérisé en ce que** l'on utilise un solvant aprotique choisi parmi des hydrocarbures halogénés en C₁-C₁₀, de préférence le chloroforme, des éthers, de préférence l'éther diéthylique et le tétrahydrofurane (THF), et leurs mélanges.

7. Procédé selon l'une des revendications 1 à 6, **caractérisé en ce que**, dans l'étape de procédé (c), on utilise comme agent d'extraction du propane avec jusqu'à 95 % en masse d'éther diméthylique (DME).

8. Procédé selon l'une des revendications 1 à 7, **caractérisé en ce que** l'extraction de l'étape (c) est conduite sous une pression comprise entre 3 et 20 MPa et à une température de 30 à 100°C.

9. Procédé selon l'une des revendications 1 à 8, **caractérisé en ce que** l'extraction est conduite en continu dans un procédé à contre-courant.

10. Procédé selon l'une des revendications 2 à 8, **caractérisé en ce que** l'on établit un gradient de température dans la colonne de séparation de manière que la température de tête soit supérieure de 5 à 50°C à celle du bas de colonne.

11. Procédé selon l'une des revendications 2 à 10, **caractérisé en ce que** l'on sépare l'agent d'extraction de la phase inférieure contenant la formulation par abaissement de la pression et/ou élévation de la température.

12. Procédé selon l'une des revendications 1 à 11, **caractérisé en ce que** l'on obtient la formulation sous forme d'une poudre coulante.
